# FASCICULE DE BREVET EUROPEEN

(11) **EP 0 745 379 B1**
(45) Date de publication et mention de la délivrance du brevet: **30.07.1997**
(21) Numéro de dépôt: 96401078.9
(22) Date de dépôt: 15.05.1996
(51) Int. Cl.: A61K 7/48, A61K 7/06, A61K 7/08, A61K 7/00, A61K 7/02

(54) **Composition solide expansée amidonné contenant des particules de polyamide sphéroidales calibrées et utilisations en application topique**
Expandiertes festes Produkt auf Basis von Stärke, das kugelähnliche bestimmte Grösse enthält sowie seiner Anwendung zur topischen Verwendung
Starch expanded solid composition containing spherial polyamide particles of a determined size and its use in topical application

(30) Priorité: 29.05.1995 FR 9506322
(43) Date de publication de la demande: 04.12.1996
(73) Titulaire: L'OREAL, 75008 Paris (FR)
(72) Inventeur: Roulier, Véronique, 75002 Paris (FR); Mellul, Myriam, 94210 L'Hay-Les-Roses (FR); Gabin, Gérard, 75008 Paris (FR); Holz, Katrin, 1012 Lausanne (CH)
(74) Mandataire: Miszputen, Laurent

(56) Documents cités:
- EP-A- 0 544 349
- WO-A-92/08759
- FR-A- 2 555 441
- DATABASE WPI Week 8643 Derwent Publications Ltd., London, GB; AN 86-282496 XP002012842 "cosmetic used in foundation cream or eye shadow-contains starch fatty acid ester(s) and sucrose derivatives" & JP-A-61 207 320 (SHISEIDO K.K.) , 13 Septembre 1986

## Description

La présente invention a trait à des compositions solides expansées dont la matrice est constituée d'un réseau alvéolaire formé à partir d'un produit riche en amidon et contient des particules de polyamide sphéroïdales calibrées ainsi qu'à leurs utilisations en application topique.

On connaît dans l'industrie alimentaire des produits expansés à base d'amidon et d'ingrédients comestibles, obtenus par extrusion dans un ou plusieurs extrudeurs mono- ou bi-vis notamment les snacks apéritifs, les chips, les corn-flakes, les produits pour petit-déjeuner à base de céréales, les biscuits.

La demanderesse a découvert de manière surprenante de nouvelles formes galéniques à usage cosmétique ou dermatologique sous la forme d'une composition solide expansée dont la matrice est constituée d'un réseau alvéolaire formé à partir d'un produit riche en amidon et contient des particules de polyamide sphéroïdales calibrées ainsi qu'à leurs utilisation en application topique.

La Demanderesse a découvert de façon inattendue que l'introduction de particules de polyamide sphéroïdales calibrées dans une matrice constituée d'un réseau alvéolaire amidonné obtenue par extrusion, permettait d'obtenir des produits expansés d'une très grande légèreté et pouvant atteindre des volumes d'expansion élevés. Une fois réduits en poudre, la Demanderesse a découvert que de tels produits constituaient des charges de maquillage d'une grande douceur, d'une grande légèreté et présentaient de bonnes propriétés de délitage.

Les compositions de l'invention par leur nouvelle structure alvéolaire amidonnée peuvent constituer de nouvelles formes galéniques solides pour le maquillage sous forme de boudins, pellets, feuilles ou flocons expansés appliqués directement sur la peau ou le visage ou être réduites en poudre et utilisées de façon classique comme poudre de maquillage.

Elles peuvent se présenter également sous forme de poudre de soin et/ou d'hygiène, appliquée directement sur la peau, le cuir chevelu ou les cheveux par exemple des shampooings à sec ou des poudres libres pour le soin du corps.

Elles peuvent également se présenter soit sous forme de poudre soit sous forme de boudins, de pellets, de feuilles ou de flocons expansés, stockés à l'état sec, et partiellement réhydratables après immersion dans un milieu aqueux pour reconstituer des produits de démaquillage ou des formulations pour le gommage doux de la peau, du corps, du visage et/ou du cou.

Les produits de gommage, encore appelés « scrub », usuels contiennent soit des particules fondantes et grasses difficilement éliminables de la peau par massage ou soit des particules insolubles constituées de charges dures qui présentent en général l'inconvénient d'être trop abrasives, d'être irritantes, de frotter la peau sans fondre et de subsister sur la peau.

Les compositions selon l'invention par leur structure alvéolaire amidonnée expansée et la présence de particules de polyamide sphéroïdales calibrées, peuvent former directement, après immersion dans l'eau, des suspensions de particules expansées, légères exerçant une action de gommage sensiblement douce et qui sont facilement éliminables par massage.

Réduites à l'état de poudre, les compositions selon l'invention peuvent également être utilisées comme phase pulvérulente dans la fabrication de produits de maquillage et en particulier des poudres compactées et conduire à des poudres plus légères, de toucher très doux et non-gras.

Les compositions selon l'invention sont des compositions solides expansées dont la matrice est constituée d'un réseau alvéolaire formé à partir d'un produit riche en amidon et contient des particules de polyamide sphéroïdales calibrées.

En règle générale, les polyamides utilisées sont répertoriées sous le nom CTFA de *"nylon 12"* ou *"nylon 6".*

Les particules de polyamide utilisées dans l'invention peuvent être celles vendues sous la dénomination "ORGASOL" par la Société ATOCHEM. Le procédé d'obtention de ces particules est par exemple celui décrit dans le document FR 2 619 385 ou dans le document EP 303 530. Ces particules de polyamide sont par ailleurs connues selon leurs différentes propriétés physico-chimiques sous le nom de *"polyamide 12"* ou *"polyamide 6".*

Les particules utilisées dans l'invention peuvent également être celles vendues sous la dénomination SP500 par la Société KOBO.

Dans les compositions selon l'invention, les particules ont une densité allant de 1g/cm³ à 1,84g/cm³ et en particulier, une densité allant de 1,0g/cm³ à 1,4g/cm³.

Les particules de l'invention sont sphériques et pleines ; elles ont notamment des dimensions moyennes allant de 5 µm à 50 µm et plus particulièrement allant de 10 µm à 30 µm.

Les particules de polyamide sont présentes dans les compositions de l'invention dans des concentrations allant, préférentiellement de 20 à 80% en poids et plus particulièrement de 30 à 70% en poids par rapport au poids total de la composition.

Les produits riches en amidon utilisés dans les compositions selon l'invention sont choisis de préférence parmi les farines de céréale telles que les farines de blé, de maïs, de riz, d'avoine, de germes de blé ou la farine de pomme de terre ; les amidons purs couramment utilisés en alimentaire tels que les amidons de maïs, de pomme de terre, de tapioca, d'avoine ; les amidons modifiés au niveau du rapport amylose/amylopectine tels que le produit HYLON VII vendu par la société AMYLUM ; les amidons modifiés par réticulation ou par un groupe fonctionnel tels que l'amidon de maïs réticulé vendu sous le nom RESISTAMYL E2 par la société AMYLUM, l'amidon de maïs faiblement quaternisé vendu sous le nom MYPLUS W7 par la société AMYLUM, l'amidon de pomme de terre vendu sous le nom SUPRAMYL P 60 par AMYLUM ou l'amidon de maïs hydroxypropylé vendu sous le nom MERIGEL EF6 par AMYLUM.

La matrice constituée du réseau alvéolaire amidonné formé à partir de ces produits riches en amidon est présente dans les compositions selon l'invention, de préférence, dans une proportion allant de 20 à 80% en poids et plus particulièrement de 30 à 70% en poids par rapport au poids total de la composition.

Les compositions selon l'invention présente une teneur en eau de préférence inférieure ou égale à 5% en poids et plus particulièrement allant de 1 à 2 % en poids par rapport au poids de la composition.

Les compositions selon l'invention peuvent contenir en plus une phase grasse. Cette phase grasse peut comprendre des huiles et/ou des cires d'origine animale, végétale, minérale ou synthétique, seules ou en mélanges.

Parmi les huiles utilisables, on peut citer l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; les huiles d'hydrocarbures, telles que les huiles de paraffine, le squalane, la vaseline; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octyldodécyle, le succinate de 2-éthyl-hexyle, le malate de diisostéaryle, le triisostéarate de glycérine ou de diglycérine; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées; les acides gras supérieurs tels que l'acide myristique, l'acide palmitique, l'acide stéarique, l'acide béhénique, l'acide oléique, l'acide linoléique, l'acide linolénique ou l'acide isostéarique; les alcools gras supérieurs tels que le cétanol, l'alcool stéarylique ou l'alcool oléique.

Parmi les cires utilisables, on peut citer les cires d'abeille, les cires de lanoline et les cires d'insectes de Chine; les cires de Carnauba, de Candelilla, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée; les paraffines, les cires microcristallines, les cires de Montan et les ozokérites; les cires de polyéthylène, les cires obtenues par la synthèse de Fisher-Tropsch, les copolymères cireux ainsi que leurs esters, et les cires de silicone telles que les polyalcoxy et polyalkylsiloxanes.

La phase grasse est présente dans des proportions allant, préférentiellement, de 0,5 à 5% en poids plus préférentiellement de 0,3 à 5% en poids par rapport au poids total de la composition.

La phase grasse peut, en outre, comprendre des additifs tels que des actifs cosmétiques lipophiles et/ou des ingrédients liposolubles généralement utilisés en cosmétique comme les parfums. De préférence, ces additifs peuvent être présents en une quantité de 0-20% par rapport au poids total de la phase grasse.

Les compositions selon l'invention peuvent contenir en plus des pigments, de préférence en une quantité de 0-50% par rapport au poids total de la composition finale. Ces pigments peuvent être choisis parmi les pigments minéraux, les pigments organiques et les pigments nacrés.

Parmi les pigments minéraux, on peut citer, par exemple le dioxyde de titane (rutile ou anatase), éventuellement traité en surface; les oxydes de fer noir, jaune, rouge et brun; le violet de manganèse; le bleu outremer; l'oxyde de chrome éventuellement hydraté ; le bleu ferrique.

Parmi les pigments organiques, on peut citer, par exemple, les pigments D & C red, D & C orange, D & C yellow, le noir de carbone, et les laques à base de carmin de cochenille.

Les pigments nacrés peuvent être choisis, notamment, parmi les pigments nacrés blancs tels que le mica recouvert d'oxyde de titane ou l'oxychlorure de bismuth; les pigments nacrés colorés tels que le micatitane avec des oxydes de fer, le micatitane avec du bleu ferrique ou de l'oxyde de chrome, le micatitane avec un pigment organique du type précité, ainsi que les pigments à base d'oxychlorure de bismuth.

Les compositions peuvent contenir également d'autres charges minérales ou organiques habituellement utilisés dans les produits de maquillage telles que, par exemple, le talc, les micas, le kaolin, la silice, les oxydes de zinc et de titane, le carbonate de calcium, le carbonate et l'hydrocarbonate de magnésium, les poudres de polymères synthétiques non expansées, les savons métalliques dérivés d'acide carboxylique en C₈-C₂₂. Elles sont présentes dans des concentrations allant préférentiellement de 0 à 50% en poids par rapport au poids de la composition.

Les compositions selon l'invention peuvent également contenir un ou plusieurs tensio-actifs non ioniques, anioniques, cationiques ou amphotères, habituellement utilisés en cosmétique. La quantité d'agent tensioactif utilisée est de préférence de 2 à 30% par rapport au poids total de la composition.

Les compositions selon l'invention selon l'invention peuvent également contenir en plus des actifs cosmétiques hydrosolubles.

Parmi les actifs cosmétiques, on peut citer les agents antioxydants ou anti-radicaux libres; les agents hydratants ou humectants tels que la glycérine et le collagène; les agents filtres UV tels que la benzophénone. Ces actifs hydrosolubles peuvent être présents dans la composition finale en une quantité de 0 à 20%, de préférence 5 à 15%. en poids.

La présente invention concerne également un procédé de préparation d'une composition telle que définie précédemment, caractérisé par le fait que celle-ci est obtenue à partir du produit riche en amidon, des particules de polyamide sphéroïdales calibrées et des constituants additionnels éventuels tels qu'indiqués ci-dessus en présence d'eau, par mélange, malaxage, expansion et extrusion dans un extrudeur bi-vis.

L'extrudeur utilisé pour le procédé de l'invention est choisi parmi les extrudeurs bi-vis tel que celui décrit dans la demande FR94-00756.

Les matières premières sont introduites, à l'entrée de l'extrudeur bi-vis, dans la zone d'alimentation à température ambiante, de préférence à environ 20°C, puis sont amenées dans la zone de transport à une température ,de préférence, à environ 50°C, puis sont malaxées et comprimées dans diverses zones de l'extrudeur maintenues à une température allant, préférentiellement, de 100 à 160°C; la masse obtenue est transportée vers la sortie de l'extrudeur et extrudée au travers d'une filière pour y subir une expansion.

Pendant la phase de mélange, le produit riche en amidon se gélatinise et forme, après extrusion, un réseau alvéolaire constituant la matrice des produits expansés finaux. Les particules de polyamide fondent partiellement et créent une matrice expansée avec la farine.

Un autre objet de l'invention consiste en de nouvelles compositions cosmétiques ou dermatologiques, caractérisées par le fait qu'elles sont constituées par une composition solide expansée telle que définie ci-dessus.

Ces compositions peuvent se présenter sous la forme de boudins, pellets ou flocons expansés ou bien être réduites à l'état de poudre. Elles peuvent être stockées à sec et partiellement réhydratables après immersion dans l'eau pour former directement une suspension aqueuse liquide ou semi-liquide de particules expansées.

Ces compositions peuvent être des produits pour le maquillage. Elles peuvent être appliquées sur le visage soit directement soit par l'intermédiaire d'un outil de maquillage tel qu'un pinceau, une houpette ou un tampon applicateur.

Les compositions selon l'invention peuvent être des produits pour le soin et/ou l'hygiène de la peau, des muqueuses, du cuir chevelu ou des cheveux.

Elles peuvent se présenter sous forme de poudre et appliquées directement sur la peau, le cuir chevelu ou les cheveux comme par exemple un shampooing sec ou une poudre libre pour le soin du corps.

Ces compositions peuvent également se présenter sous forme de poudre ou bien de boudins, pellets ou flocons expansés, stockés à sec et partiellement réhydratables après immersion dans l'eau pour former une composition de démaquillage ou une composition de gommage doux de la peau, du corps, du visage et/ou du cou.

Bien entendu, l'homme de l'art veillera à choisir un ou des composés complémentaires éventuels et/ou leurs quantités de manière telle que les propriétés avantageuses attachés intrinsèquement à la composition conforme à l'invention ne soient pas, ou substantiellement pas, altérées par la ou les adjonctions envisagées.

Les exemples qui suivent servent à illustrer l'invention sans pour autant en limiter la portée.

### EXEMPLES

### Exemple 1 Gommage doux sous forme de poudre à réhydrater

Le produit final a pour formulation suivante :

| | |
|---|---|
| - Farine de blé | 35,0% en poids |
| - Particules de polyamide sphéroïdales calibrées vendues sous le nom ORGASOL par la société ATOCHEM. | 50,0% en poids |
| - Silice vendue sous le nom SB700 par la Société MAPRECOS | 15,0% en poids |

### MODE OPERATOIRE :

Les pellets sont obtenus par extrusion dans un extrudeur bi-vis. Les matières premières sont introduites à l'entrée de l'extrudeur à une température de 30°C. Elles sont ensuite amenées dans la zone de transport à une température de 50°C , puis sont malaxées et comprimées dans diverses zones de l'extrudeur maintenues à 120°C. La masse ainsi malaxée et comprimée, est transportée vers la sortie de l'extrudeur et extrudée au travers d'une filière de 5 mm de diamètre. La vitesse de rotation des vis est de 500 tours/minute. Les boudins obtenus à la sortie de la filière sont réduits à l'état de poudre au moyen d'un broyeur à broches classique, placé à la sortie de l'extrudeur. Le produit sous forme de poudre forme une composition de gommage doux aprés réhydratation dans l'eau, au moment de l'utilisation.

### Exemple 2 Démaquillant moussant sous forme de pellets à réhydrater

Le produit final a pour formulation suivante :

| | |
|---|---|
| - Farine de blé | 40,0% en poids |
| - Particules de polyamide sphéroïdales calibrées vendues sous le nom ORGASOL par la société ATOCHEM. | 40,0% en poids |
| - Talc | 10,0% en poids |
| - Lauryl-éther sulfate de sodium | 10,0% en poids |

### MODE OPERATOIRE :

Les pellets sont obtenus par extrusion dans un extrudeur bi-vis. Les matières premières sont introduites à l'entrée de l'extrudeur à une température de 30°C. Elles sont ensuite amenées dans la zone de transport à une température de 50°C, puis sont malaxées et comprimées dans diverses zones de l'extrudeur maintenues à 120°C. La masse ainsi malaxée et comprimée, est transportée vers la sortie de l'extrudeur et extrudée au travers d'une filière de 5 mm de diamètre. La vitesse de rotation des vis est de 500 tours/minute. Les boudins obtenus à la sortie de la filière sont réduits sous formes de pellets de 3mm de diamètre au moyen d'un couteau granulateur en sortie de l'extrudeur. Le produit sous forme de poudre forme une composition de démaquillage aprés réhydratation dans l'eau, au moment de l'utilisation.

### EXEMPLE COMPARATIF

On réalise des boudins expansés ayant la formulation suivante :

### Formulation A :

| | |
|---|---|
| - Farine de blé | 80,0% en poids |
| - Talc | 10,0% en poids |
| - Lauryl-éther sulfate de sodium | 10,0% en poids |

On réalise également des boudins expansés selon l'invention ayant la formulation suivante :

### Formulation B:

| | |
|---|---|
| - Farine de blé | 40,0% en poids |
| - Particules de polyamide sphéroïdales calibrées vendues sous le nom ORGASOL par la société ATOCHEM. | 40,0% en poids |
| - Talc | 10,0% en poids |
| - Lauryl-éther sulfate de sodium | 10,0% en poids |

Les boudins expansés de formulation A ou B sont fabriqués selon le même mode opératoire que l'exemple 2, le diamètre de la filière à la sortie de l'extrudeur est de 5 mm.
Le diamètre moyen de la section d'un boudin de formulation A est de 8-10mm tandis que celui d'un boudin expansé de formulation B est de 14-16mm. Les boudins expansés selon l'invention ont donc un taux d'expansion sensiblement plus élevé que celui des boudins ne comportant pas les particules de polyamide.

## Revendications

1. Composition solide expansée dont la matrice est constituée d'un réseau alvéolaire formé à partir d'un produit riche en amidon et contenant au moins des particules de polyamide sphéroïdales calibrées.

2. Composition cosmétique selon la revendication 1, caractérisée par le fait que les particules ont une densité allant de 1g/cm³ à 1,84 g/cm³.

3. Composition cosmétique selon l'une quelconque des revendications 1 et 2, caractérisée par le fait que les particules ont une densité allant de 1 g/cm³ à 1,4 g/cm³.

4. Composition cosmétique selon l'une quelconque des revendications 1 à 3, caractérisée par le fait que les particules ont une granulométrie allant de 5 µm à 50 µm.

5. Composition selon l'une quelconque des revendications 1 à 4, caractérisée par le fait qu'elle contient de 20 à 80% en poids de particules de polyamide sphéroïdales calibrées par rapport au poids total de la composition.

6. Composition selon l'une quelconque des revendications 1 à 5, caractérisée par le fait que le produit riche en amidon est choisi parmi les farines de céréale ou de pomme de terre ; les amidons purs ; les amidons modifiés au niveau du rapport amylose/amylopectine ;les amidons réticulés et les amidons modifiés par un groupe fonctionnel.

7. Composition selon l'une quelconque des revendications 1 à 6, dans laquelle la matrice constituée d'un réseau alvéolaire amidonné représente de 20 à 80% en poids du poids total de la composition.

8. Composition selon l'une quelconque des revendications 1 à 7, dans laquelle la teneur en eau est inférieure à 5% en poids par rapport au poids total de la composition.

9. Composition selon la revendication 8, dans laquelle la teneur en eau varie de 1 à 2% en poids par rapport au poids total de la composition.

10. Composition selon l'une quelconque des revendications 1 à 9, caractérisée par le fait qu'elle contient en plus une phase grasse.

11. Composition selon la revendication 10, comprenant 0,5 à 5% en poids de phase grasse.

12. Composition selon l'une des revendications 10 et 11, dans laquelle la phase grasse comprend des huiles et/ou des cires d'origine animale, végétale, minérale ou synthétique, seules ou en mélanges.

13. Composition selon l'une quelconque des revendication 1 à 12, caractérisée par le fait qu'elle contient des adjuvants choisis parmi les pigments, des charges minérales ou organiques, les tensio-actifs, les actifs liposolubles, les additifs liposolubles habituellement utilisés en cosmétique, les anti-oxydants, les anti-radicaux libres, les hydratants, les humectants, les filtres solaires.

14. Procédé de préparation de la composition telle que définie dans l'une quelconque des revendications 1 à 13, caractérisée par le fait qu'elle est obtenue à partir du produit riche en amidon, des particules de polyamide sphéroïdales calibrées et des autres composants éventuels en présence d'eau, par mélange, malaxage, expansion et extrusion dans un extrudeur bi-vis.

15. Procédé selon la revendication 14, caractérisé par le fait que les matières premières sont introduites, à l'entrée de l'extrudeur, à température ambiante puis sont amenées dans la zone de transport à température d'environ 50°C, puis sont malaxées et comprimées dans diverses zones de l'extrudeur maintenues à une température allant de 100 à 160°C ; la masse obtenue est transportée vers la sortie de l'extrudeur et extrudée au travers d'une filière pour y subir une expansion.

16. Composition à usage cosmétique ou dermatologique, caractérisée par le fait qu'elle est constituée par une composition solide expansée telle que définie selon l'une quelconque des revendications 1 à 13.

17. Composition selon la revendication 16, caractérisée par le fait qu'elle se présente sous forme de boudin, pellet, feuille ou flocon expansé ou bien qu'elle est réduite à l'état de poudre.

18. Composition selon l'une quelconque des revendications 16 et 17, caractérisée par le fait qu'elle est un produit pour le maquillage.

19. Composition selon l'une quelconque des revendications 16 et 17, caractérisée par le fait qu'elle est un produit pour le soin et/ou l'hygiène de la peau, des muqueuses, du cuir chevelu ou des cheveux.

20. Composition selon l'une quelconque des revendications 16 et 17, caractérisée par le fait qu'elle est partiellement réhydratable après immersion dans l'eau et susceptible de reconstituer une suspension de particules expansées.

21. Composition selon la revendication 20, caractérisée par le fait qu' elle est un produit de gommage de la peau, du corps, du visage et/ou du cou ou un produit de démaquillage.

22. Utilisation d'une composition selon l'une quelconque des revendications 1 à 13 dans et pour la fabrication d'une composition à usage cosmétique ou dermatologique.

23. Utilisation comme phase pulvérulente d'une composition selon l'une quelconque des revendications 1 à 13 réduite à l'état de poudre, dans et pour la fabrication d'une composition pour le maquillage.

## Claims

1. Expanded solid composition whose matrix consists of a cellular network formed from a starch-rich product and which contains at least some size-graded spheroidal polyamide particles.

2. Cosmetic composition according to Claim 1, characterized in that the particles have a density ranging from 1 g/cm³ to 1.84 g/cm³.

3. Cosmetic composition according to either of Claims 1 and 2, characterized in that the particles have a density ranging from 1 g/cm³ to 1.4 g/cm³.

4. Cosmetic composition according to any one of Claims 1 to 3, characterized in that the particles have a particle size ranging from 5 µm to 50 µm.

5. Composition according to any one of Claims 1 to 4, characterized in that it contains from 20 to 80 % by weight of size-graded spheroidal polyamide particles relative to the total weight of the composition.

6. Composition according to any one of Claims 1 to 5, characterized in that the starch-rich product is chosen from cereal or potato flours; pure starches; starches modified in respect of the amylose/amylopectin ratio; and crosslinked starches and starches modified with a functional group.

7. Composition according to any one of Claims 1 to 6, in which the matrix consisting of a starch-based cellular network represents from 20 to 80 % by weight of the total weight of the composition.

8. Composition according to any one of Claims 1 to 7, in which the water content is less than 5 % by weight relative to the total weight of the composition.

9. Composition according to Claim 8, in which the water content varies from 1 to 2 % by weight relative to the total weight of the composition.

10. Composition according to any one of Claims 1 to 9, characterized in that it contains, in addition, a fatty phase.

11. Composition according to Claim 10, comprising 0.5 to 5 % by weight of fatty phase.

12. Composition according to either of Claims 10 and 11, in which the fatty phase comprises oils and/or waxes of animal, vegetable, mineral or synthetic origin, alone or mixed.

13. Composition according to any one of Claims 1 to 12, characterized in that it contains adjuvants chosen from pigments, inorganic or organic fillers, surfactants, fat-soluble active agents, fat-soluble additives customarily used in cosmetics, antioxidants, free-radical scavengers, hydrating agents, humectants and sunscreen agents.

14. Process for preparing the composition as defined in any one of Claims 1 to 13, characterized in that it is obtained from the starch-rich product, size-graded spheroidal polyamide particles and other possible components in the presence of water, by mixing, kneading, expansion and extrusion in a twin-screw extruder.

15. Process according to Claim 14, characterized in that the starting materials are introduced at the entry of the extruder at room temperature, are then conveyed to the transport zone at a temperature of approximately 50°C and are then kneaded and compressed in various zones of the extruder which are maintained at a temperature ranging from 100 to 160°C; the mass obtained is transported to the exit of the extruder and extruded through a die to undergo an expansion thereat.

16. Composition for cosmetic or dermatological use, characterized in that it consists of an expanded solid composition as defined according to any one of Claims 1 to 13.

17. Composition according to Claim 16, characterized in that it takes the form of an expanded cylinder, pellet, leaf or flake, or in that it is alternatively reduced to the powder state.

18. Composition according to either of Claims 16 and 17, characterized in that it is a make-up product.

19. Composition according to either of Claims 16 and 17, characterized in that it is a product for the care and/or hygiene of the skin, mucosae, scalp or hair.

20. Composition according to either of Claims 16 and 17, characterized in that it can be partially rehydrated after immersion in water and is capable of reconstituting a suspension of expanded particles.

21. Composition according to Claim 20, characterized in that it is an exfoliating product for the skin, body, face and/or neck, or a make-up removal product.

22. Use of a composition according to any one of Claims 1 to 13, in and for the manufacture of a composition for cosmetic or dermatological use.

23. Use as pulverulent phase of a composition according to any one of Claims 1 to 13 reduced to the powder state, in and for the manufacture of a make-up composition.

## Patentansprüche

1. Expandierte, feste Zusammensetzung, deren Matrix aus einem porigen Netzwerk besteht, das aus einem stärkereichen Produkt gebildet ist, und die mindestens kalibrierte kugelförmige oder kugelähnliche Polyamidpartikel enthält.

2. Kosmetische Zusammensetzung nach Anspruch 1, dadurch gekennzeichnet, daß die Partikel eine Dichte von 1 bis 1,84 g/cm³ aufweisen.

3. Kosmetische Zusammensetzung nach einem der Ansprüche 1 und 2, dadurch gekennzeichnet, daß die Partikel eine Dichte von 1 bis 1,4 g/cm³ aufweisen.

4. Kosmetische Zusammensetzung nach einem der Ansprüche 1 bis 3, dadurch gekennzeichnet, daß die Partikel eine Korngröße von 5 bis 50 µm aufweisen.

5. Zusammensetzung nach einem der Ansprüche 1 bis 4, dadurch gekennzeichnet, daß sie 20 bis 80 Gew.-% kalibrierte kugelförmige Polyamidpartikel, bezogen auf das Gesamtgewicht der Zusammensetzung, enthält.

6. Zusammensetzung nach einem der Ansprüche 1 bis 5, dadurch gekennzeichnet, daß das stärkereiche Produkt unter Getreidemehlen und Kartoffelmehl, reinen Stärken, hinsichtlich des Amylose/Amylopectin-Verhältnisses modifizierten Stärken, vernetzten Stärken und durch ein funktionelle Gruppe modifizierten Stärken ausgewählt ist.

7. Zusammensetzung nach einem der Ansprüche 1 bis 6, wobei die aus einem stärkehaltigen porigen Netzwerk gebildete Matrix 20 bis 80 Gew.-% des Gesamtgewichts der Zusammensetzung ausmacht.

8. Zusammensetzung nach einem der Ansprüche 1 bis 7, wobei der Wassergehalt weniger als 5 Gew.-%, bezogen auf das Gesamtgewicht der Zusammensetzung, beträgt.

9. Zusammensetzung nach Anspruch 8, wobei der Wassergehalt im Bereich von 1 bis 2 Gew.-%, bezogen auf das Gesamtgewicht der Zusammensetzung, variiert.

10. Zusammensetzung nach einem der Ansprüche 1 bis 9, dadurch gekennzeichnet, daß sie außerdem eine Fettphase enthält.

11. Zusammensetzung nach Anspruch 10, die 0,5 bis 5 Gew.-% Fettphase enthält.

12. Zusammensetzung nach einem der Ansprüche 10 und 11, wobei die Fettphase Öle und/oder Wachse tierischer, pflanzlicher, anorganischer oder synthetischer Herkunft, einzeln oder in Form von Gemischen, umfaßt.

13. Zusammensetzung nach einem der Ansprüche 1 bis 12, dadurch gekennzeichnet, daß sie Hilfsstoffe enthält, die unter Pigmenten, anorganischen und organischen Füllstoffen, grenzflächenaktiven Stoffen, fettlöslichen Wirkstoffen, fettlöslichen Zusätzen, die üblicherweise in der Kosmetik verwendet werden, Antioxidantien, Mitteln gegen freie Radikale, Hydratisierungsmitteln, Feuchthaltemitteln und Lichtschutzfiltern ausgewählt sind.

14. Verfahren zur Herstellung der Zusammensetzung, wie sie in einem der Ansprüche 1 bis 13 definiert ist, dadurch gekennzeichnet, daß sie aus einem stärkereichen Produkt, kalibrierten kugelförmigen Polyamidpartikeln und weiteren möglichen Bestandteilen in Gegenwart von Wasser durch Mischen, Kneten, Expandieren und Extrudieren in einem Doppelschneckenextruder hergestellt wird.

15. Verfahren nach Anspruch 14, dadurch gekennzeichnet, daß die Ausgangsmaterialien am Einlaß des Extruders bei Umgebungstemperatur zugegeben werden, anschießend in einer Förderzone auf eine Temperatur von etwa 50 °C gebracht werden, wonach sie in verschiedenen Zonen des Extruders, die bei einer Temperatur von 100 bis 160 °C gehalten werden, geknetet und komprimiert werden und wonach die erhaltene Masse zum Auslaß des Extruders gefördert wird und durch eine Düse extrudiert wird, wo sie expandiert wird.

16. Zusammensetzung für die kosmetische oder dermatologische Verwendung, dadurch gekennzeichnet, daß sie aus einer expandierten festen Zusammensetzung besteht, wie sie in einem der Ansprüche 1 bis 13 definiert ist.

17. Zusammensetzung nach Anspruch 16, dadurch gekennzeichnet, daß sie in Form von expandierten Strängen, Pellets, Blättchen oder Flocken vorliegt oder daß sie zu einem Pulver zerkleinert ist.

18. Zusammensetzung nach einem der Ansprüche 16 und 17, dadurch gekennzeichnet, daß es sich um ein Schminkprodukt handelt.

19. Zusammensetzung nach einem der Ansprüche 16 und 17, dadurch gekennzeichnet, daß es sich um ein Produkt für die Pflege und/oder Hygiene der Haut, der Schleimhäute, der Kopfhaut oder der Haare handelt.

20. Zusammensetzung nach einem der Ansprüche 16 und 17, dadurch gekennzeichnet, daß sie nach Eintauchen in Wasser teilweise rehydratisierbar ist und geeignet ist, eine Suspension expandierter Partikel zu bilden.

21. Zusammensetzung nach Anspruch 20, dadurch gekennzeichnet, daß sie ein Produkt für die abradierende Behandlung der Haut, des Körpers, des Gesichts und/oder des Halses oder eine Anschminkprodukt ist.

22. Verwendung einer Zusammensetzung nach einem der Ansprüche 1 bis 13 in der und für die Herstellung einer Zusammensetzung für die kosmetische oder dermatologische Verwendung.

23. Verwendung als pulverförmige Phase einer Zusammensetzung nach einem der Ansprüche 1 bis 13, die zu einem Pulver zerkleinert ist, in der und für die Herstellung einer Schminkzusammensetzung.
